# EUROPÄISCHE PATENTSCHRIFT

(11) **EP 1 263 470 B1**
(45) Veröffentlichungstag und Bekanntmachung des Hinweises auf die Patenterteilung: **25.06.2003**
(21) Anmeldenummer: 01900427.4
(22) Anmeldetag: 11.01.2001
(51) Int. Cl.: A61K 47/48, C08B 37/16

(54) **VERFAHREN ZUR HERSTELLUNG EINES KOENZYM Q10 / $g(g)-CYCLODEXTRIN KOMPLEXES**
METHOD FOR PRODUCING A COENZYME Q10 / $g(g)-CYCLODEXTRIN COMPLEX
PROCEDE POUR LA PRODUCTION D'UN COMPLEXE COENZYME Q10 - $g(g)-CYCLODEXTRINE

(30) Priorität: 27.01.2000 DE 10003493
(43) Veröffentlichungstag der Anmeldung: 11.12.2002
(73) Patentinhaber: Wacker-Chemie GmbH, 81737 München (DE)
(72) Erfinder: CULLY, Jan, 84518 Garching/Alz (DE); MOLDENHAUER, Jens, 84489 Burghausen (DE)
(74) Vertreter: Potten, Holger
(86) Internationale Anmeldenummer: EP0100305
(87) Internationale Veröffentlichungsnummer: WO01054730

(56) Entgegenhaltungen:
- EP-A- 0 875 240
- MOTWANI, M. UND ZATZ, J.L.: "Applications of cyclodextrins in skin products" COSMETICS AND TOILETRIES, Bd. 112, Nr. 7, Juli 1997 (1997-07), Seiten 39-47, XP000984079
- LOUKAS, Y.L. ET AL.: "Novel liposome-based multicomponent systems for the protection of photolabile agents" INTERNATIONAL JOURNAL OF PHARMACEUTICS, Bd. 117, Nr. 1, 1995, Seiten 85-94, XP000986334
- MATSUBARA, K. ET AL.: "Controlled release of the LHRH agonist buserelin acetate from injectable suspensions containing triacetylated cyclodextrins in an oil vehicle" JOURNAL OF CONTROLLED RELEASE, Bd. 31, Nr. 2, 1994, Seiten 173-180, XP000458317

## Beschreibung

Die Erfindung betrifft ein Verfahren zur Herstellung eines Koenzym Q10 / γ-Cyclodextrin Komplexes.

Cyclodextrine sind cyclische Oligosaccharide, die aus 6, 7 oder 8 α(1-4)-verknüpften Anhydroglukoseeinheiten aufgebaut sind. Die beispielsweise durch enzymatische Stärkekonversion hergestellten α-, β- oder γ-Cyclodextrine unterscheiden sich in dem Durchmesser ihrer hydrophoben Kavität und eignen sich generell zum Einschluß zahlreicher lipophiler Substanzen.

Koenzym Q10 (Ubichinon), ist eine endozelluläre Komponente, die eine wichtige Rolle bei dem mitochondrialen Elektrontransport spielt. Seit bekannt ist, daß Koenzym Q10 die Atmungskette der Zelle verbessert und die mitochondriale Membran stärkt, wird Koenzym Q10 bei der Behandlung von Herzerkrankungen eingesetzt. Koenzym Q10 wirkt auch als Fänger von freien Radikalen. Es wird ferner bei der Behandlung von degenerativen Erkrankungen genutzt. Weiter verbessert Koenzym Q10 die Freisetzung von Energie im Körper. Diese Eigenschaft wird von der "dietary supplement" Industrie bei der Herstellung von Präparaten für Sportler und Gewichtsabnahme genutzt. Koenzym Q10 ist zudem die aktive Komponente in verschiedenen kosmetischen Formulierungen.

Koenzym Q10 ist eine fettlösliche Substanz. Sie ist ein gelbliches Pulver und an Luft instabil. Sie ist sehr schlecht wasserlöslich. Eine Formulierung mit γ-Cyclodextrin ist luftund lichtstabil und erhöht die Löslichkeit in Wasser sowie die Bioverfügbarkeit.

In Journal Acta Poloniae Pharmaceutica, (1995), Vol. 52, No 5, S. 379 - 386 und (1996), Vol. 53, No 3, S. 193 - 196 wird die Komplexierung von Koenzym Q10 mit verschiedenen Cyclodextrinen und Cyclodextrinderivaten mit einer "Kneading" und einer "Heating" Methode beschrieben. Die dort beschriebenen Verfahren zur Herstellung eines γ-Cyclodextrin Komplexes mit Koenzym Q10 weisen im Produktionsmaßstab verschiedene Nachteile auf.

Bei Verwendung der "Kneading" Methode im Produktionsmaßstab ist die für die Komplexierung notwendige Verteilung des Koenzyms Q10 erst nach mehrstündigem Rühren des Ansatzes gewährleistet. Bei der "Heating" Methode wird das Material in einem geschlossenem Gefäß über 164 Stunden bei 333 K gehalten.
Aus den wirtschaftlichen Gründen sind diese Methoden für die Herstellung eines γ-Cyclodextrin/Koenzym Q10 Komplexes im Produktionsmaßtab daher nicht verwendbar.

Aufgabe der Erfindung ist es, ein Verfahren zur Verfügung zu stellen, welches die Nachteile des Standes der Technik nicht aufweist und das eine schnelle, unkomplizierte Herstellung eines γ-Cyclodextrin/Koenzym Q10 Komplexes ermöglicht.

Die Aufgabe wird gelöst durch ein Verfahren, bei dem eine Mischung aus γ-Cyclodextrin und Koenzym Q10 durch Homogenisierung und Energiezufuhr behandelt wird.

Vorzugsweise wird im erfindungsgemäßen Verfahren eine wäßrige Mischung aus γ-Cyclodextrin und Koenzym Q10 eingesetzt.

Das Verfahren geht beispielsweise von einer konzentrierten, wäßrigen γ-CD-Lösung aus, der das Koenzym Q10 zugesetzt wird. Es ist jedoch ebenso möglich, zunächst eine wäßrige Koenzym Q10 Suspension herzustellen und dieser γ-CD zuzusetzen.

Die Mischung enthält vorzugsweise eine γ-CD-Konzentration zwischen 10 - 30 Gewichts-%. Besonders bevorzugt ist eine CD-Konzentration von 16 - 25 Gew.%.

Das Gewichts-Verhältnis Koenzym Q10 zu γ-CD liegt vorzugsweise zwischen 1 : 1 und 1 : 100, besonders bevorzugt zwischen 1 : 1 und 1 : 8, insbesondere bevorzugt zwischen 1 : 1 und 1 : 6.

Die Homogenisierung erfolgt beispielsweise mittels Ultraturrax, Ultraschallgerät, Hochdruckhomogenisator, Kugelmühle oder anderer zur Homogenisierung üblicher Geräte.

Sie erfolgt vorteilhaft mittels solcher Geräte, die gleichzeitig auch die Energiezufuhr im Mikrobereich gewährleisten. Dies ist z. B. mittels eines Ultraschall erzeugenden Gerätes oder einer Kugelmühle möglich.

Die Energiezufuhr kann beispielsweise durch Erwärmen der wäßrigen Phase, oder der Mischung aus γ-Cyclodextrin und Koenzym Q10 vor oder während der Homogenisierung erfolgen.

Es ist jedoch ebenso möglich, in der Regel ausreichend und bevorzugt, nur die bei der Homogenisierung ebenfalls anfallende Wärme, wie z. B. die durch den Ultraschall oder die in der Kugelmühle erzeugte Wärme, zu nutzen.

Vorteilhafterweise erfolgt daher die Energiezufuhr durch Geräte, die gleichzeitig auch eine Homogenisierung bewirken.

Es hat sich als besonders vorteilhaft erwiesen, die Komplexierung in einer Kugelmühle durchzuführen, da es dabei gleichzeitig mit der Mikronisierung der Koenzym Q10 Teilchen auch zu einer lokalen Verschmelzung dieser Teilchen kommen kann, wobei anschließend die Bildung der Komplexe mit dem aufgelösten gamma CD erfolgt.

Besonders bevorzugt erfolgen Homogenisierung und Energiezufuhr gleichzeitig, z. B. durch den Einsatz einer Kugelmühle.

Beispielsweise wird die Koenzym Q10 und γ-CD/γ-CD-Lösung in eine Kugelmühle gegeben und 10 bis 60 min bei 25 °C unter Sticks'toff gemischt.

Die kinetische Energie der Kugeln mikronisiert beim Aufprall die Koenzym Q10 Teilchen weiter. Ein Teil dieser Energie wird in die Wärme umgewandelt, diese schmilzt die in der Mikroumgebung sich befindlichen Q10 Teilchen lokal. Diese werden dann von dem umgebenen CD komplexiert.

Das erfindungsgemäße Verfahren erfolgt vorzugsweise in einem Temperaturbereich von 10 - 80 °C.

Besonders bevorzugt wird bei 15 - 40 °C, insbesondere bevorzugt bei etwa 25 °C gearbeitet.

Die Homogenisierungsdauer hängt von der Temperatur, der Rührgeschwindigkeit und der Geometrie der Kugelmühle ab. In der Regel ist eine Mischzeit von 1 bis 60 Min. ausreichend.

Die Romplexierung erfolgt in der Regel unter Normaldruck. Dies gilt natürlich nicht bei der Durchführung des Verfahrens unter Verwendung von Geräten (z. B. eines Hochdruckemulgators), die üblicherweise bei anderen Druckbedingungen zum Einsatz kommen. Bei Verfahren unter Einsatz solcher Geräte kommen die für das jeweilige Gerät üblichen Druckbedingungen zum Einsatz.

Bevorzugt findet die Komplexierung unter Schutzgasatmosphäre (Stickstoff oder Argon) statt.

Die Komplexe können direkt in Form der Reaktionsmischung verwendet werden.

Sie werden jedoch vorzugsweise durch Filtration, Zentrifugation, Trocknung, Mahlen, Sieben, Sichten, Granulieren, Tablettieren entsprechend der jeweils üblichen Vorgehensweise isoliert und zu einem stabilen Pulver aufbereitet.

So kann die Trocknung des Koenzym Q10 / γ-CD Komplexes beispielsweise im Trockenschrank bei 55 °C 12 Stunden lang erfolgen und sich an die Trocknung eine Vermahlung anschließen.

Die folgenden Beispiele dienen der weiteren Erläuterung der Erfindung.

### Beispiel 1:

18 g γ-CD (käuflich erhältlich unter der Bezeichnung Cavamax bei Firma Wacker-Chemie, München) wurden zu 72 ml Wasser in eine Kugelmühle PE 75 der Firma Netzsch mit Mahlkörpern d 0,4 mm aus Zirkoniumoxid gegeben und 30 Sekunden bei 1000 UPM und Raumtemperatur resuspendiert. Dann wurde 4,5 g Koenzym Q10 Pulver (erhältlich von der Firma Eurotech GmbH) zugegeben und 15 Minuten bei der Raumtemperatur bei 1200 UPM weiter gemischt.
Nach der Abtrennung der Zirkoniumoxidmahlkörper mit einem Sieb wurde das Material bei 55 °C im Trockenschrank getrocknet.
Das so erhaltene weiße Pulver mit einem Wassergehalt von ca 6 % beinhaltet 22 % Koenzym Q10.

### Beispiel 2:

15g γ-CD wurden im 75 ml Wasser bei 70 °C aufgelöst. Zu der Lösung wurden 3g Koenzym Q10 im 6 ml Diethylether gegeben, anschließend wurde 30 Sekunden mit einem IKA Ultraturrax T50 bei 10 000 UPM homogenisiert. Das Gemisch wurde auf dem Magnetrührer innerhalb 30 Minuten auf Raumtemperatur abgekühlt. Anschließend wurde das Material bei 55° C im Trockenschrank getrocknet.
Das so erhaltene weiße Pulver mit einem Wassergehalt von ca 6 % beinhaltet 20 % Koenzym Q10.

### Beispiel 3:

6 g Koenzym Q10 wurden in 60 ml Wasser bei 70 °C mit einem Ultraturrax homogenisiert. Zu dem Homogenat wurden 80 ml einer 70° C warmen wässerigen Lösung enthaltend 26 g γ-CD gegeben. Anschließend wurde das Gemisch mit einem IKA Ultraturrax T50 bei 10000 UPM 30 Sekunden homogenisiert. Das Gemisch wurde auf einem Magnetrührer mittels Rühren innerhalb 30 Minuten auf die Raumtemperatur abgekühlt. Dann wurde das Material bei 55° C im Trockehschrank getrocknet. Das weiße Pulver mit einem Wassergehalt von ca. 6% beinhaltet 25 % Koenzym Q10.

## Patentansprüche

1. Verfahren zur Herstellung eines γ-Cyclodextrin/Koenzym Q10 Komplexes **dadurch gekennzeichnet, daß** eine Mischung aus γ-Cyclodextrin und Koenzym Q10 durch Homogenisierung und Energiezufuhr behandelt wird.

2. Verfahren gemäß Anspruch 1, **dadurch gekennzeichnet, daß** eine wäßrige Mischung aus γ-Cyclodextrin und Koenzym Q10 eingesetzt wird.

3. Verfahren gemäß Anspruch 1 oder 2, **dadurch gekennzeichnet, daß** die Mischung eine γ-CD-Konzentration zwischen 10 - 30 Gewichts-% enthält.

4. Verfahren gemäß einem der Ansprüche 1 bis 3, **dadurch gekennzeichnet, daß** das Gewichts-Verhältnis Koenzym Q10 zu γ―CD zwischen 1 : 1 und 1 : 100 liegt.

5. Verfahren gemäß einem der Ansprüche 1 bis 4, **dadurch gekennzeichnet, daß** die Homogenisierung mittels Ultraschallgerät, Kugelmühle, Ultraturrax, Hochdruckhomogenisator oder anderer zur Homogenisierung üblicher Geräte erfolgt.

6. Verfahren gemäß einem der Ansprüche 1 bis 5, **dadurch gekennzeichnet, daß** die Energiezufuhr durch Erwärmen der Mischung aus γ-Cyclodextrin und Koenzym Q10 vor oder während der Homogenisierung erfolgt.

7. Verfahren gemäß einem der Ansprüche 1 bis 6, **dadurch gekennzeichnet, daß** die Energiezufuhr durch Geräte, die gleichzeitig auch eine Homogenisierung bewirken, erfolgt.

8. Verfahren gemäß Anspruch 7, **dadurch gekennzeichnet, daß** Homogenisierung und Energiezufuhr gleichzeitig durch den Einsatz einer Kugelmühle erfolgt.

9. Verfahren gemäß einem der Ansprüche 1 bis 8, **dadurch gekennzeichnet, daß** die Homogenisierung im Temperaturbereich von 10 bis 80 °C durchgeführt wird.

10. Verfahren gemäß einem der Ansprüche 1 bis 9, **dadurch gekennzeichnet, daß** die Verfahrensdauer zwischen 1 und 60 min liegt.

## Claims

1. Process for preparing a γ-cyclodextrin/coenzyme Q10 complex, **characterized in that** a mixture of γ-cyclodextrin and coenzyme Q10 is treated by homogenization and energy input.

2. Process according to Claim 1, **characterized in that** an aqueous mixture of γ-cyclodextrin and coenzyme Q10 is employed.

3. Process according to Claim 1 or 2, **characterized in that** the γ-CD concentration in the mixture is between 10-30% by weight.

4. Process according to any of Claims 1 to 3, **characterized in that** the ratio of coenzyme Q10 to γ-CD by weight is between 1:1 and 1:100.

5. Process according to any of Claims 1 to 4, **characterized in that** the homogenization is effected by ultrasound apparatus, ball mill, Ultraturrax, high-pressure homogenizer or other equipment usual for homogenization.

6. Process according to any of Claims 1 to 5, **characterized in that** the energy input is effected by heating the mixture of γ-cyclodextrin and coenzyme Q10 before or during the homogenization.

7. Process according to any of Claims 1 to 6, **characterized in that** the energy input is effected by equipment which simultaneously also brings about a homogenization.

8. Process according to Claim 7, **characterized in that** homogenization and energy input are effected simultaneously through use of a ball mill.

9. Process according to any of Claims 1 to 8, **characterized in that** the homogenization is carried out in the temperature range from 10 to 80°C.

10. Process according to any of Claims 1 to 9, **characterized in that** the process takes between 1 and 60 min.

## Revendications

1. Procédé pour la préparation d'un complexe γ-cyclodextrine/coenzyme Q10, **caractérisé en ce qu'**on traite par homogénéisation et apport d'énergie un mélange de γ-cyclodextrine et coenzyme Q10.

2. Procédé selon la revendication 1, **caractérisé en ce qu'**on utilise un mélange aqueux de γ-cyclodextrine (γ-CD) et coenzyme Q10.

3. Procédé selon la revendication 1 ou 2, **caractérisé en ce que** le mélange contient une concentration de γ-CD comprise entre 10 et 30 % en poids.

4. Procédé selon l'une quelconque des revendications 1 à 3, **caractérisé en ce que** le rapport pondéral de la coenzyme Q10 à γ-CD est compris entre 1:1 et 1:100.

5. Procédé selon l'une quelconque des revendications 1 à 4, **caractérisé en ce que** l'homogénéisation s'effectue au moyen d'un appareil à ultrasons, d'un broyeur à billes, d'un homogénéisateur Ultraturrax, d'un homogénéisateur à haute pression ou d'autres appareils usuels pour l'homogénéisation.

6. Procédé selon l'une quelconque des revendications 1 à 5, **caractérisé en ce que** l'apport d'énergie s'effectue par chauffage du mélange de γ-cyclodextrine et coenzyme Q10 avant ou pendant l'homogénéisation.

7. Procédé selon l'une quelconque des revendications 1 à 6, **caractérisé en ce que** l'apport d'énergie s'effectue au moyen d'appareils qui effectuent simultanément également une homogénéisation.

8. Procédé selon la revendication 7, **caractérisé en ce que** l'homogénéisation et l'apport d'énergie s'effectuent simultanément au moyen d'un broyeur à billes.

9. Procédé selon l'une quelconque des revendications 1 à 8, **caractérisé en ce que** l'homogénéisation est effectuée dans la plage de températures allant de 10 à 80°C.

10. Procédé selon l'une quelconque des revendications 1 à 9, **caractérisé en ce que** la durée du processus est comprise entre 1 et 60 minutes.
